Europäisches Patentamt

European Patent Office

Office européen des brevets

(19)

(11) Veröffentlichungsnummer : **0 422 572 B1**

(12) **EUROPÄISCHE PATENTSCHRIFT**

(45) Veröffentlichungstag der Patentschrift :
07.06.95 Patentblatt 95/23

(51) Int. Cl.$^6$ : **G01N 33/18,** G01N 27/06

(21) Anmeldenummer : **90119301.1**

(22) Anmeldetag : **09.10.90**

(54) **Verfahren zur kontinuierlichen Bestimmung von Dimethylformamid und Dimethylamin in wässrigen Lösungen, insbesondere in Abwässern.**

(30) Priorität : **12.10.89 DE 3934025**

(43) Veröffentlichungstag der Anmeldung :
**17.04.91 Patentblatt 91/16**

(45) Bekanntmachung des Hinweises auf die
Patenterteilung :
**07.06.95 Patentblatt 95/23**

(84) Benannte Vertragsstaaten :
**DE ES FR GB GR IT NL**

(56) Entgegenhaltungen :
**EP-A- 0 096 754**
**GB-A- 1 517 000**
**US-A- 2 386 927**
**SYNTHESEFASERN, Verlag Chemie, 1981; v.**
**FALKAI, S. 204/**
**WPI, Woche 01, 1976, Derwent Publications**
**Ltd., London (GB); AN 76-00240X (01)/**

(73) Patentinhaber : **HOECHST**
**AKTIENGESELLSCHAFT**
**D-65926 Frankfurt (DE)**

(72) Erfinder : **Gruteser, Rudolf**
**Nelkenstrasse 15**
**W-8420 Kelkheim (DE)**

**Beschreibung**

Die vorliegende Erfindung betrifft ein Verfahren zur quantitativen Bestimmung von Dimethylformamid bzw. Dimethylamin in wäßrigen Lösungen, insbesondere in kontinuierlich anfallenden, wie z.B. in Abwässern. Wäßrige Lösungen, insbesondere Abwässer, die geringe Mengen von Dimethylformamid und Dimethylamin enthalten, fallen in vielen Bereichen der Technik an.

Aus der EP-A-96,754 ist ein Verfahren zur kontinuierlichen Aufbereitung von Abwasser bekannt. In der GB-A-1,517,000 wird ein Verfahren zur Qualitätskontrolle von Wässern beschrieben, die beim Betrieb von Kraftwerken anfallen. Eine Bestimmung von Dimethylformamid bzw. Dimethylamin wird dort nicht beschrieben.

Dimethylformamid ist aufgrund seiner kleinen Molekülgröße, seiner Dielektrizitätskonstante, seiner Elektronendonator-Eigenschaften und seiner Fähigkeit Komplexe zu bilden, als Lösungsmittel besonders für hochmolekulare Verbindungen sehr gut geeignet. Es ist daher eines der wenigen Lösungsmittel, das zur Herstellung verspinnbarer Polyacrylnitrillösungen geeignet ist (vergl. Falkai, Synthesefasern, S. 204, Verlag Chemie, 1981), aber auch in der Kunstleder- und Beschichtungsindustrie bei der Herstellung von Polyurethansystemen für qualitativ hochwertige Oberflächen ist man auf die Verwendung von Dimethylformamid als Lösungsmittel angewiesen. Auch bestimmte Polyamide, insbesondere vollaromatische Polyamide, können nur in Form ihrer Lösungen in DMF oder ähnlichen Lösungsmitteln versponnen werden.

Wegen seines selektiven Lösevermögens für bestimmte Kohlenwasserstoffe und Gase wird Dimethylformamid bei einer Reihe großtechnischer Prozesse verwendet. So läßt sich z.B. Acetylen auch aus acetylenarmen Spaltgasen vorteilhaft mit DMF extrahieren, wobei gleichzeitig acetylenfreies Ethylen erhalten werden kann.

Seit einigen Jahren wird DMF sogar als selektives Absorptionsmittel für Butadien aus der $C_4$-Fraktion von Crackgasen verwendet (vergl. US-A-2,386,927). In der Erdölindustrie dient es als selektives Extraktionsmittel für Aromaten aus Kohlenwasserstoffgemischen, sowie zur Entschwefelung von Dieselölen und Schmierölen, aber auch in der Pharmaindustrie bedient man sich der hervorragenden Lösungs- und Extraktionseigenschaften des DMF für Vitamine, Hormone, Sulfonamide und Antibiotika.

DMF stellt somit in einer großen Anzahl großtechnischer Prozesse einen unersetzlichen Hilfsstoff dar.

Bei Störungen im Betriebsablauf oder auch bei bestimmten in Grenzbereichen liegenden Betriebszuständen dieser Prozesse kann Dimethylformamid oder sein Hydrolyseprodukt Dimethylamin in mehr oder weniger kleinen Mengen in die Abwässer dieser Produktionsprozesse gelangen und muß dann aus ökologischen Gründen daraus entfernt werden. Diese Notwendigkeit setzt aber die möglichst lückenlose Überwachung der Dimethylformamid- und Dimethylamin-Konzentration in den Betriebsabwässern voraus. Zu diesem Zweck bedarf es einer möglichst einfach zu handhabenden, und schnelle Ergebnisse liefernden Analysenmethode zur Bestimmung von Dimethylformamid und Dimethylamin in wäßrigen Lösungen.

Bisher hat sich als spezifische Methode für die Dimethylformamid-Bestimmung in wäßrigen Lösungen besonders die Hydrolyse mit Säure bewährt. Das gebildete Dimethylamin wird anschließend aus alkalischer Lösung destillativ abgetrennt, nitrosiert und die entstandenen Nitrosoverbindung polarographisch bestimmt. Diese Analysenmethode ist zwar relativ genau, jedoch zu zeitraubend und umständlich für eine ständige Kontrolle von Betriebsabwässern.

Eine weitere gängige Methode zum Nachweis oder auch zur Reinheitsprüfung von Dimethylformamid ist z.B. die Gaschromatographie. Auch diese Methode setzt eine häufige Entnahme von Einzelproben und deren Analyse voraus und eignet sich daher schlecht für eine ständige Abwasserkontrolle. Ein weiteres bekanntes Verfahren, welches eine niedrige Nachweisgrenze hat, einfach anzuwenden ist und schnelle und genaue Resultate liefert basiert auf der Messung der elektrischen Leitfähigkeit von Dimethylaminhaltigen wäßrigen Lösungen. Aber auch dieses Meßprinzip ist bisher nur zur diskontinuierlichen Bestimmung der Dimethylamin-Konzentration in wäßrigen Lösungen eingesetzt worden. Die herkömmliche Probenvorbereitung erfordert das Vorliegen von Einzelproben und setzt voraus, daß andere, den elektrischen Strom leitende Stoffe fehlen.

Es wurde nun überraschenderweise gefunden, daß die elektrische Leitfähigkeitsmessung sich auch als Meßprinzip für ein kontinuierliches Verfahren zur Bestimmung der Dimethylformamid- und Dimethylaminkonzentrationen in wäßrigen Lösungen, insbesondere in Betriebsabwässern einsetzen läßt, wenn eine Kombination von Verfahrensbedingungen innerhalb bestimmter Grenzen eingehalten wird.

Die vorliegende Erfindung betrifft somit ein Verfahren zur kontinuierlichen Bestimmung von Dimethylformamid und/oder von Dimethylamin in wäßrigen Lösungen, insbesondere in Betriebsabwässern, welches darin besteht, daß man dem Abwasserstrom kontinuierlich einen konstanten Teilstrom entnimmt, daß man den Teilstrom unter konstanten Bedingungen mit einer mit konstanter Geschwindigkeit zudosierten Alkalilösung hydrolysiert und einer Teilverdampfung unter konstanten Verdampfungsbedingungen unterwirft, den dabei erhaltenen Dampfstrom durch Kühlung kondensiert, im Kondensat in einer Durchflußmeßzelle kontinuierlich die elektrische Leitfähigkeit bestimmt, und die Meßwerte, z.B. anhand einer Eichkurve, in die gewünschten Konzentrationen umrechnet.

Um sicherzustellen, daß reproduzierbare Meßergebnisse erhalten werden, ist es von Bedeutung, bestimmte Verfahrensparameter innerhalb enger, einmal gewählter Grenzen konstant zu halten. So ist es zweckmäßig, die Hydrolyse des Teilstromes bei einer zwischen 70 und 100°C, vorzugsweise zwischen 85 und 95°C gewählten konstanten Temperatur auszuführen. Um sicherzustellen, daß diese Temperatur tatsächlich vom Beginn der Hydrolyse an bis zu ihrem Ende konstant eingehalten wird, ist es besonders vorteilhaft, den vom Abwasserstrom abgezweigten Teilstrom und zweckmäßigerweise auch die dem Teilstrom zudosierte Alkalilösung auf die gewünschte Hydrolysetemperatur vorzuwärmen.

Alkalilösungen, die für die Hydrolyse eingesetzt werden können, sind nicht auf basisch reagierende Verbindungen der Alkalimetalle beschränkt, sondern es können im Prinzip alle Lösungen, insbesondere wäßrige Lösungen, hinreichend starker Basen eingesetzt werden. Aus ökonomischen und ökologischen Gründen ist es besonders zweckmäßig, als Alkalilösungen wäßrige Lösungen basisch reagierender Alkalisalze, insbesondere wäßrige Alkalihydroxidlösungen einzusetzen. Besonders bevorzugt sind hierzu Lösungen von Natrium- oder Kaliumhydroxid, insbesondere solche mit einer hohen Konzentration der Alkaliverbindung.

Die Alkalilösung wird dem konstanten Abwasserteilstrom mit konstanter Geschwindigkeit in einer solchen Menge zudosiert, daß in der Mischung eine konstante Alkalikonzentration im Bereich von 0,1 bis 2,5, vorzugsweise 0,25 bis 1,25, insbesondere 0,5 bis 0,9 g-Äquivalente pro 1 ergibt.

Dabei ist unter der Alkalikonzentration die rechnerische Alkalikonzentration zu verstehen, die sich ergibt, wenn die zudosierte Alkalimenge mit einer der Teilstrommenge gleichen Menge reinen Wassers gemischt würde.

Wie bereits oben angegeben, soll eine Teilverdampfung des alkalisch gestellten Abwasserteilstroms unter konstanten Bedingungen durchgeführt werden. Zur Einstellung dieser konstanten Verdampfungbedingungen wird zweckmäßigerweise die Verdampfung in einem Verdampfungsgefäß durchgeführt, in dem mit konstanter Geschwindigkeit gerührt wird und dessen Füllstand konstant gehalten wird. Auch die Heizleistung des Verdampfers wird zweckmäßigerweise innerhalb einmal gewählter enger Grenzen konstant gehalten.

Der Füllstand des Verdampfers wird auf konstanter Höhe gehalten, indem man bei der, durch die konstante Wärmezufuhr bedingten konstanten Destillationsgeschwindigkeit, am Boden des Verdampfungsgefäßes den Verdampferinhalt mit konstanter, geregelter Geschwindigkeit abzieht.

Das bei der Verdampfung und Kondensation des Hydrolysats erhaltene Kondensat wird zur Messung seiner elektrischen Leitfähigkeit zweckmäßigerweise einer bekannten Durchflußmeßzelle bei einer konstanten zwischen 5 und 50°C liegenden Temperatur zugeführt. Die Meßzelle selbst wird dabei zweckmäßigerweise thermostatisiert. Anstelle einer sehr genauen Temperaturkonstanz bei der elektrischen Leitfähigkeitsmessung oder ergänzend hierzu, kann die Meßzelle mit einer temperaturkompensierten Leitfähigkeitsmeßkette bestückt werden.

Die elektrische Leitfähigkeit L des Destillats hängt von der darin vorliegenden Dimethylamin-Konzentration c und einer durch die Meßanordnung bestimmten Konstante K ab:
$$L = L' (c,K)$$
Führt man alle Messungen mit einer einmal gewählten Meßanordnung durch, so vereinfacht sich der Zusammenhang zu
$$L = L' (c).$$
Dieser Zusammenhang wird vorteilhafterweise für die zu messende Substanz, hier also Dimethylamin, durch eine Eichkurve einmal bestimmt.

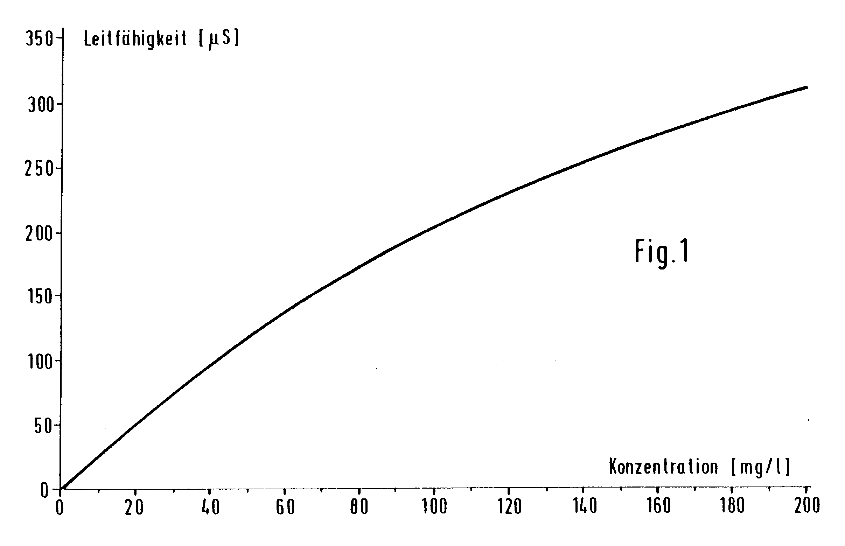

Die Figur 1 veranschaulicht eine solche Eichkurve, die den Zusammenhang zwischen der in der Meßzelle gemessenen Leitfähigkeit in Mikrosiemens ($\mu$S) und der Dimethylamin-Konzentration des die Meßzelle durchlaufenden Destillats wiedergibt.

Bei der Auswertung der Leitfähigkeitsmeßwerte ist man aber keineswegs auf die Benutzung einer Eichkurve beschränkt, wenngleich dies die einfachste Möglichkeit ist. Alternativ kann auch eine, die Eichkurve repräsentierende, aus den Punkten der Kurve abgeleitete, mathematische Funktion zur Umrechnung der Leitfähigkeitswerte auf Konzentrationswerte herangezogen werden. Beim Einsatz eines elektronischen Rechners können die Meßwerte dann sogleich als Konzentrationswerte ausgegeben werden. Auch eine Berechnung der Konzentration aus der gemessenen Äquivalenz-Leitfähigkeit G und der Widerstandskapazität der Meßzelle unter Zuhilfenahme bekannter einfacher empirischer Gleichungen, wie sie beispielsweise in Houben-Weyl, "Methoden der Organischen Chemie", (1955) Bd. 3, "Physikalische Methoden", Teil 2, Seite 8, zu entnehmen sind, ist selbstverständlich möglich, sofern die in den Gleichungen enthaltenen empirischen Stoffkonstanten einmal für das zu messende Dimethylamin bestimmt worden sind.

Die vorliegende Erfindung betrifft auch eine Vorrichtung zur Durchführung des oben beschriebenen Analysenverfahrens. Die erfindungsgemäße Vorrichtung besteht aus einer Dosierpumpe (2) mit saugseitig angeschlossenem Einlauf (1), für den dem Hauptstrom der zu analysierenden Flüssigkeit zu entnehmenden Teilstrom, einem druckseitig an die Dosierpumpe (2) angeschlossenen, auf konstanter Hydrolysetemperatur gehaltenen, Vorwärmaggregat (3), einer am Ausgang dieses Aggregats angeschlossenen Mischstrecke (4), in die ein Einlauf (5) einmündet, an welchen über die Dosierpumpe (6) ein regelbar beheizbares und auf konstante Temperatur einstellbares Vorratsgefäß (7) für die Alkalilösung angeschlossen ist, einem am Auslauf der Mischstrecke angeschlossenen, mit leistungsgeregelten Rühr- (8) und Heiz- (9) Vorrichtungen versehenen Verdampfer (10) der einen Bodenauslauf (11) mit einem regelbaren Abflußventil (12) aufweist, welches den geregelten Abfluß des Verdampferinhaltes gestattet und mit einem Dampfauslaß (13), einem an dem Dampfauslaß angeschlossenen absteigenden Kühler (14) und der an dessen Auslauf angeschlossenen Durchflußmeßzelle (15), sowie der dazugehörenden Anzeige und Recheneinheit (16).

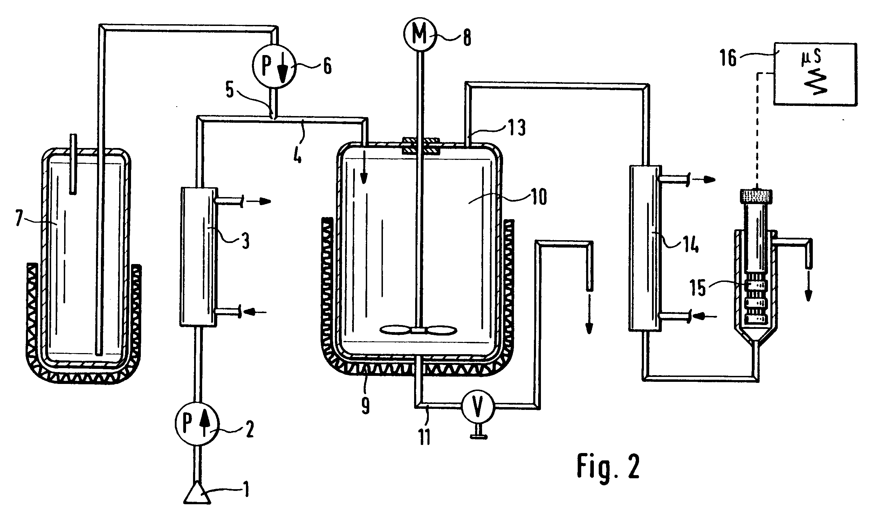

Die Figur 2 zeigt schematisch den Aufbau der oben beschriebenen erfindungsgemäßen Meßvorrichtung.

Die in dem erfindungsgemäßen Meßaufbau vorhandenen Dosierpumpen können im Prinzip jedem bekannten Typ angehören, solange das Pumpenmaterial durch die zu fördernden Flüssigkeiten nicht oder nicht nennenswert angegriffen wird. Besonders bewährt im praktischen Einsatz haben sich regelbare Schlauchpumpen.

Auch das in den Bodenauslauf des Verdampfers eingebaute, geregelte Abflußventil kann von jeder bekannten Konstruktion sein, insbesondere können aber auch hier Dosierpumpen, insbesondere Schlauchpumpen zur Regelung des Abflusses eingesetzt werden.

Unter dem an den Dampfauslaß des Verdampfers angeschlossenen absteigenden Kühler ist ein Kühler zu verstehen, bei dem das Kondensat nicht mehr in den Kessel zurückläuft, sondern quantitativ der daran angeschlossenen Durchfluß-Leitfähigkeitsmeßzelle zugeführt wird.

Es hat sich als besonders vorteilhaft erwiesen, den Verdampfer (10) oder eine damit kommunizierende Röhre mit einem Füllstandsfühler auszurüsten. Dieser Fühler kann für den Füllstand einen Analogwert oder aber einen Grenzwert ausgeben. Dieser Füllstandsmeßfühler wird zweckmäßigerweise mit den Dosierpumpen (2) und (6), dem Abflußregler am Bodenauslauf des Verdampfers und dem Verdampferheizelementes des Verdampfers so verschaltet, daß durch das Füllstandssignal die Leistung dieser Bauelemente beeinflußt werden können im Sinne einer Konstanhaltung des Füllstands.

Für spezielle Meßaufgaben kann es auch vorteilhaft sein, zwischen den Verdampfer und den absteigenden Kühler einen Rektifikationsaufsatz einzuschalten. Ein solcher Aufsatz kann auch gewünschtenfalls an mehreren Stellen mit Anschlüssen für die Abnahme verschiedener Fraktionen versehen sein, die durch getrennte absteigende Kühler und getrennte, daran angeschlossene elektrische Leitfähigkeitsmeßzellen ausgewertet werden.

Das erfindungsgemäße Verfahren eignet sich hervorragend zur kontinuierlichen Bestimmung auch geringer Konzentrationen von Dimethylformamid oder Dimethylamin in wäßrigen Flüssigkeiten, insbesondere in Betriebsabwässern.

Ein besonderer Vorteil des erfindungsgemäßen Meßverfahrens besteht darin, daß seine Ergebnisse nicht durch die Anwesenheit von Salzen oder anderen starken Elektrolyten in den auszumessenden Flüssigkeiten gestört werden. Bei der Durchführung des Verfahrens ist lediglich darauf zu achten, daß für den Fall, daß die zu messenden wäßrigen Lösungen größere Mengen Kohlensäure enthalten, die im Verfahren zudosierte Alkalimenge so bemessen wird, daß auch die Kohlensäure in Form von Carbonaten im Verdampfer zurückgehalten wird. Ein weiterer entscheidender Vorteil des erfindungsgemäßen Meßverfahrens liegt darin, daß jede Änderung der Dimethylformamid- oder Dimethylamin-Konzentration in den zu messenden wäßrigen Flüssigkeiten mit einer sehr kurzen Zeitverschiebung angezeigt wird. Dies bedeutet, daß man aufgrund der Meßergebnisse eine sehr effektive Regelung der Bedingungen der Produktionsverfahren, aus denen die gemessenen Abwässer stammen, durchführen kann.

Das erfindungsgemäße Verfahren, das oben im wesentlichen zur Bestimmung von Dimethylformamid und Dimethylamin in wäßrigen Lösungen beschrieben worden ist, kann selbstverständlich leicht unter Anpassung der Hydrolysebedingungen zur Konzentrationsbestimmung anderer wasserlöslicher Säureamide und Amine in wäßrigen Lösungen eingesetzt werden, soweit die entsprechenden Amine ausreichend wasserdampfflüchtig sind.

## Patentansprüche

1. Verfahren zur kontinuierlichen Bestimmung von Dimethylformamid und/oder Dimethylamin in wäßrigen Lösungen, insbesondere in Betriebsabwässern, dadurch gekennzeichnet, daß man dem Abwasserstrom kontinuierlich einen konstanten Teilstrom entnimmt, den Teilstrom bei einer zwischen 70 und 100°C liegenden konstanten Temperatur mit einer, mit konstanter Geschwindigkeit zudosierten Alkalilösung alkalisch hydrolysiert, wobei die Alkalilösung dem Teilstrom in einer Menge zudosiert wird, die in der Mischung eine konstante Alkalikonzentration im Bereich von 0,1 bis 2,5 g-Äquivalente pro l ergibt, den Teilstrom einer Teilverdampfung in einem Verdampfungsgefäß unterwirft, wobei in dem Verdampfungsgefäß mit konstanter Geschwindigkeit gerührt wird der Füllstand konstant gehalten wird, den dabei erhaltenen Dampfstrom durch Kühlung kondensiert, im Kondensat in einer Durchflußmeßzelle kontinuierlich die elektrische Leitfähigkeit bestimmt und die erhaltenen Meßwerte anhand einer Eichkurve oder bekannter Formeln in die gewünschten Konzentrationswerte umrechnet.

2. Verfahren nach Anspruch 1, dadurch gekennzeichnet, daß der alkalische Teilstrom anschließend einer Teilverdampfung unterworfen wird.

3. Verfahren nach mindestens einem der Ansprüche 1 und 2, dadurch gekennzeichnet, daß die Hydrolysetemperatur durch Vorwärmen des Teilstroms und der Alkalilösung vor deren Vereinigung eingestellt wird.

4. Verfahren gemäß mindestens einem der Ansprüche 1 bis 3, dadurch gekennzeichnet, daß die konstanter Geschwindigkeit zudosierte Alkalilösung eine Alkalihydroxidlösung ist.

5. Verfahren gemäß mindestens einem der Ansprüche 1 bis 4, dadurch gekennzeichnet, daß zur Einstellung konstanter Verdampfungsbedingungen die Heizleistung des Verdampfers konstant gehalten wird.

6. Verfahren gemäß mindestens einem der Ansprüche 1 bis 5, dadurch gekennzeichnet, daß das Kondensat der Durchflußmeßzelle mit einer konstanten zwischen 5 und 50°C liegenden Temperatur zugeführt wird und die Meßzelle thermostatisiert ist.

7. Verfahren gemäß mindestens einem der Ansprüche 1 bis 6, dadurch gekennzeichnet, daß die elektrische Leitfähigkeit mittels einer temperaturkompensierten Leitfähigkeitsmeßkette gemessen wird.

8. Vorrichtung zur Durchführung des Verfahrens des Anspruchs 1, bestehend aus einer Dosierpumpe (2) mit saugseitig angeschlossenem Einlauf (1) für den, dem Hauptstrom der zu analysierenden Flüssigkeit zu entnehmenden Teilstrom, einem druckseitig an die Dosierpumpe (2) angeschlossenen, auf konstanter Hydrolysetemperatur gehaltenen Vorwärmaggregat (3), einer am Ausgang des Vorwärmaggregats (3) angeschlossenen Mischstrecke (4), in die ein Einlauf (5) einmündet, an welchen über die Dosierpumpe (6) ein regelbar beheizbares und auf konstante Temperatur einstellbares Vorratsgefäß (7) für Alkalilösung angeschlossen ist, mit leistungsgeregelten Rühr- (8) und Heiz- (9) Vorrichtungen versehenen Verdampfer (10) mit einem Bodenauslauf (11) mit geregeltem Abflußventil (12) und mit einem Dampfauslaß (13), einem an den Dampfauslaß angeschlossenen absteigenden Kühler (14) und der an dessen Auslauf angeschlossenen Durchflußmeßzelle (15) sowie der dazugehörenden Anzeige- und Recheneinheit (16).

9. Vorrichtung gemäß Anspruch 8, dadurch gekennzeichnet, daß der Verdampfer (10) oder ein damit kommunizierendes Rohr mit einem Füllstandsfühler ausgerüstet ist.

10. Vorrichtung gemäß mindestens einem der Ansprüche 8 und 9, dadurch gekennzeichnet, daß die Dosierpumpen (2) und (6), der Abflußregler an dem Bodenauslauf des Verdampfers und das Heizelement des Verdampfers mit dem Füllstandsfühler so verschaltet sind, daß durch das Füllstandssignal die Leistung dieser Bauelemente beeinflußt werden kann im Sinne einer Konstanthaltung des Füllstands.

11. Vorrichtung gemäß mindestens einem der Ansprüche 8 bis 10, dadurch gekennzeichnet, daß zwischen Verdampfer (10) und absteigendem Kühler (14) ein Rektifikationsaufsatz geschaltet ist.

**Claims**

1. A method for the continuous determination of dimethylformamide and/or dimethylamine in aqueous solutions, especially in plant effluents, which comprises continuously taking a constant part stream from the effluent stream, hydrolyzing the part stream under alkaline conditions at a constant temperature which is between 70 and 100°C by means of an alkali solution added at a constant rate, the alkali solution being added to the part stream at a rate which results in a constant alkali concentration in the range from 0.1 to 2.5 g-equivalents per 1 in the mixture, subjecting the part stream to partial vaporization in a vaporizing vessel, stirring being carried out at constant speed in the vaporization vessel and the level being kept constant, condensing the resulting vapor stream by cooling, determining the electrical conductivity in the condensate continuously in a flow cell and converting the measured values obtained into the desired concentration values by reference to a calibration curve or known equations.

2. The method as claimed in claim 1, wherein the alkaline part stream is subsequently subjected to partial vaporization.

3. The method as claimed in at least one of claims 1 and 2, wherein the hydrolysis temperature is adjusted by preheating the part stream and the alkali solution before they are combined.

4. The method as claimed in at least one of claims 1 to 3, wherein the alkali solution added at a constant rate is an alkali metal hydroxide solution.

5. The method as claimed in at least one of claims 1 to 4, wherein, to establish constant vaporization conditions, the heat output of the vaporizer is kept constant.

6. The method as claimed in at least one of claims 1 to 5, wherein the condensate is fed to the flow cell at a constant temperature which is between 5 and 50°C and the measuring cell is thermostatically controlled.

7. The method as claimed in at least one of claims 1 to 6, wherein the electrical conductivity is measured by means of a temperature-compensated conductivity measurement circuit.

8. An apparatus for carrying out the method of claim 1, comprising a metering pump (2) with an inlet (1), connected to the suction side, for the part stream to be taken from the main stream of the liquid which is to be analyzed, a preheating unit (3) connected to the delivery side of the metering pump (2) and held at a constant hydrolysis temperature, a mixing section (4) which is connected to the outlet of the preheating unit (3) and into which an inlet (5) leads to which an alkali solution stock vessel (7), which can be heated under control and is adjustable to a constant temperature, is connected via the metering pump (6), a vaporizer (10) provided with power-controlled stirring (8) and heating (9) devices and having a bottom outlet (11) with a controlled outflow valve (12) and having a vapor outlet (13), a descending condenser (14) connected to the vapor outlet and the flow cell (15) connected to the outlet thereof, and also the associated indicating and computing unit (16).

9. The apparatus as claimed in claim 8, wherein the vaporizer (10) or a tube communicating with it is fitted with a level sensor.

10. The apparatus as claimed in at least one of claims 8 and 9, wherein the metering pumps (2) and (6), the outflow controller at the bottom outlet of the vaporizer and the heating element of the vaporizer are interconnected to the level sensor in such a way that the output of these components can be influenced by the level signal in the direction of keeping the level constant.

11. The apparatus as claimed in at least one of claims 8 to 10, wherein a rectifying head is inserted between the vaporizer (10) and the descending condenser (14).

## Revendications

1. Procédé de détermination en continu du diméthylformamide et/ou de la diméthylamine en solutions aqueuses, en particulier dans des eaux résiduaires d'exploitation, caractérisé en ce qu'on prélève en continu un courant partiel constant à partir du flux des eaux résiduaires, on hydrolyse le courant partiel avec une solution alcaline introduite progressivement avec une vitesse constante, à une température constante se situant entre 70 et 100 °C, la solution alcaline étant introduite dans le courant partiel en une quantité telle qu'une concentration alcaline constante dans le mélange s'établisse dans une zone comprise entre 0,1 et 2,5 g-équivalent par 1., on soumet le courant partiel à une évaporation partielle dans un récipient d'évaporation, en agitant le contenu du récipient d'évaporation à une vitesse constante et en maintenant constant le niveau, on concentre le courant de vapeur ainsi obtenu dans un réfrigérant, on détermine en continu la conductivité électrique du condensat à l'aide d'une cellule de mesure de passage de courant et on transforme par le calcul la valeur mesurée obtenue à partir d'une courbe d'étalonnage ou à partir de formules connues en les valeurs de concentration désirées.

2. Procédé selon la revendication 1, caractérisé en ce qu'on soumet le courant partiel alcalin à une évaporation partielle ultérieure.

3. Procédé selon au moins l'une des revendications 1 et 2, caractérisé en ce que la température d'hydrolyse est établie par un chauffage préalable du courant partiel et de la solution alcaline avant leur combinaison.

4. Procédé selon au moins l'une des revendications 1 à 3, caractérisé en ce que la solution alcaline introduite progressivement avec une vitesse constante est une solution d'un hydroxyde alcalin.

5. Procédé selon au moins l'une des revendications 1 à 4, caractérisé en ce qu'on maintient constante la puissance de chauffage de l'évaporateur afin d'établir des conditions d'évaporation constantes.

6. Procédé selon au moins l'une des revendications 1 à 5, caractérisé en ce que le condensat est conduit vers la cellule de mesure de passage de courant avec une température constante se situant entre 5 et 50 °C et la cellule de mesure est thermostatée.

7. Procédé selon au moins l'une des revendications 1 à 6, caractérisé en ce que la conductivité électrique est mesurée au moyen d'une chaîne de mesure de conductivité à température compensée.

8. Dispositif pour réaliser le procédé de la revendication 1, consistant en une pompe doseuse (2) avec une entrée (1) raccordée du côté de l'admission, pour le courant partiel à prélever du flux principal du liquide à analyser, un dispositif de préchauffage (3), maintenue à une température d'hydrolyse constante, connecté du côté de refoulement de la pompe doseuse (2) un espace de mélange (4) connecté à la sortie du groupe de préchauffage (3), dans lequel débouche une entrée (5), sur laquelle est connecté un récipient (7) pour la solution alcaline, récipient que l'on peut chauffer de manière régulée et auquel on peut appliquer une température constante, un évaporateur (10) muni d'un dispositif d'agitation (8) et de chauffage (9), de puissance réglable, avec une sortie (11) à sa base munie d'une soupape de trop-plein (12) régulée et avec un échappement de vapeur (13), un réfrigérant descendant (14) connecté à l'échappement de vapeur et la cellule de mesure de passage de courant (15) connecté à cette sortie de même qu'un calculateur (16) et une unité d'affichage en faisant partie.

9. Dispositif selon la revendication 8, caractérisé en ce que l'évaporateur (10) ou un tube communiquant avec lui est muni d'une sonde à niveau.

10. Dispositif selon au moins une des revendications 8 et 9, caractérisé en ce que les pompes doseuses (2) et (6), le régulateur de niveau à la partie basse de l'évaporateur et l'élément de chauffage de l'évaporateur sont connectés avec la sonde de niveau, de telle sorte que la puissance des éléments de construction puisse être influencée par le signal de niveau de manière à maintenir constant le niveau.

11. Dispositif selon au moins une des revendications 8 à 10, caractérisé en ce qu'une soupape de rectification soit connectée entre l'évaporateur (10) et le réfrigérant descendant (14).

Fig.1

Fig. 2

EP 0 422 572 B1